# DEMANDE DE BREVET EUROPEEN

(11) **EP 2 077 278 A1**
(43) Date de publication de la demande: **08.07.2009**
(21) Numéro de dépôt: 07291602.6
(22) Date de dépôt: 21.12.2007
(51) Int. Cl.: C07K 14/705, C07K 7/08, C12N 15/12, A61K 38/10, A61K 38/17, A61P 25/24

(54) **Peptide dérivé du récepteur 3 de la neurotensine et utilisation dans le traitemement de maladies psychiatriques**

(71) Demandeur: Centre National de la Recherche Scientifique (CNRS), 75794 Paris Cedex 16 (FR)
(72) Inventeur: Mazella, Jean, 06410 Biot (FR); Petrault, Olivier, 59000 Lille (FR); Borsotto, Marc, 06130 Grasse (FR); Heurteaux, Catherine, 06600 Antibes (FR); Widmann, Catherine, 06480 La Colle sur Loup (FR)
(74) Mandataire: Novagraaf IP

(57) **Abrégé**

La présente invention se rapporte à un peptide dérivé du récepteur de la neurotensine 3 (NSTR3) et à son utilisation dans le traitement de maladies psychiatriques. La présente invention se rapporte en particulier à l'utilisation de ce peptide pour la fabrication d'un médicament, par exemple un antidépresseur. Le peptide de l'invention est **caractérisé en ce que** sa séquence est la SEQ ID n°1 annexée.

La présente invention trouve une application dans les secteurs de l'industrie pharmaceutique et notamment dans les domaines de développement du médicament dans le traitement des maladies psychiatriques. La présente invention trouve en particulier une application dans le développement d'un nouvel antidépresseur. Elle trouve par exemple également une application dans le traitement de la douleur et des processus inflammatoires.

## Description

### Domaine technique de l'invention

La présente invention se rapporte à un peptide dérivé du récepteur 3 de la neurotensine (NSTR3) et à son utilisation dans le traitement de maladies psychiatriques. La présente invention se rapporte en particulier à l'utilisation de ce peptide pour la fabrication d'un médicament, par exemple un antidépresseur.

La présente invention trouve une application dans les secteurs de l'industrie pharmaceutique et notamment dans les domaines de développement du médicament utilisé dans le traitement des maladies psychiatriques.

La présente invention trouve en particulier une application dans le développement d'un nouvel antidépresseur. Elle trouve également une application dans le traitement de la douleur.

Dans la description ci-dessous, les références entre parenthèses **(x)** renvoient à la liste des références à la fin des exemples.

### Art antérieur

Les maladies psychiatriques représentent un réel problème de Santé Publique. Les travaux les plus récents ont confirmé la forte prévalence de la dépression : sur leur vie entière, 20% des femmes et 10% des hommes ont fait, font ou feront un épisode dépressif **(1).** De tels chiffres sont à l'évidence marquants; ils le sont plus encore lorsqu'on s'intéresse à la complication majeure de la dépression, le suicide, qui se chiffre à 12000 décès par an dans des pays comme la France **(2).**

La dépression est une maladie très fréquente et souvent handicapante. Elle peut toucher jusqu'à 20% de la population dans les pays industrialisés. Ses origines sont diverses et multiples. Cette pathologie affecte aussi bien le psychisme que le comportement et la physiologie des patients. Les traitements de la dépression sont également multiples et les mécanismes d'action des médicaments utilisés ne sont pas clairement établis.

L'organisation mondiale de la santé (OMS) prévoit que la dépression unipolaire sera la deuxième cause de handicap en 2020. A la souffrance personnelle et familiale que représente la dépression s'ajoute le poids social important de cette pathologie. La dépression représente déjà l'une des premières causes d'arrêt du travail, avec une charge économique qui s'élève à plus de 30 milliards d'euros par an. Malgré l'arsenal thérapeutique mis à disposition du corps médical, en particulier les SSRI (« *selective serotonin reuptake inhibitors* ») et SNRI (« *serotonin norepinephrine reuptake inhibitors* »), 30% de la population dépressive n'a pas de traitement. Par ailleurs, le délai d'action des antidépresseurs est de l'ordre de 3 à 6 semaines et les effets secondaires sont souvent importants.

D'une façon générale, on estime que 15% des patients déprimés décèdent par suicide. Chez la plupart des malades, la dépression est due à l'interaction entre une prédisposition génétique et des facteurs environnementaux comme le stress ou les traumatismes émotionnels (3). La maladie est fréquente et le marché des antidépresseurs (AD) est immense (au moins 10 milliards d'euros par an).

Néanmoins, si ces antidépresseurs améliorent l'état des patients dans environ 70% des cas, ils n'entraînent une rémission complète de la maladie que chez 30 à 40% d'entre eux. De plus, près d'un tiers des sujets traités résiste aux traitements existants. Cet état de fait oblige donc à envisager de nouveaux traitements, capables de prendre en compte les mécanismes de la dépression **(3).**

Dans l'arsenal thérapeutique mis à disposition du corps médical, les antidépresseurs tricycliques (TCA) avec l'amitriptyline et l'imipramine ont été les premiers découverts, suivis par les inhibiteurs de la monoamine oxydase (IMAO), irréversibles et non sélectifs comme la phénelzine et la pargyline. Les effets indésirables, en particulier la cardiotoxicité des TCA (surtout en cas de surdosage) et les crises hypertensives des IMAO (interactions avec la tyramine alimentaire, le fameux « *cheese effect* ») ont poussé la recherche vers de nouvelles molécules d'efficacité thérapeutique identique mais de meilleure acceptabilité.

La notion de sélectivité est alors apparue avec les inhibiteurs spécifiques de la recapture de la noradrénaline (NA) ou de la sérotonine (5-hydroxytryptamine ou 5HT). Les essais cliniques de phase III ont démontré pour ces nouvelles molécules une efficacité équivalente aux antidépresseurs de première génération et une meilleure sécurité, notamment en cas de surdosage.

Les inhibiteurs spécifiques de la recapture de la sérotonine (ISRS) et les inhibiteurs spécifiques de la recapture de la noradrénaline (ISRN) sont actuellement les molécules les plus utilisées **(4-5).** Les AD sont ainsi le plus souvent associés à une facilitation de la transmission des systèmes monoaminergiques.

Bien que la sérotonine, la noradrénaline et la dopamine sont impliquées de façon certaine, il est admis aujourd'hui que les modifications des taux de monoamines produites par les AD et les processus adaptatifs qui en découlent, en particulier l'altération de la sensibilité de certains de leurs récepteurs, ne peuvent expliquer à eux seuls le mécanisme d'action des antidépresseurs.

Ainsi, il est difficile de corréler le délai de 3 à 6 semaines nécessaire à l'obtention des AD avec l'augmentation des taux synaptiques de monoamines, qui intervient dès la première administration du produit. En près d'un demi-siècle, le nombre d'hypothèses sur la pathogénèse de la dépression et son traitement n'a cessé d'évoluer.

Par exemple, des concentrations élevées de glucocorticoïdes sont généralement associées à un effet négatif de l'humeur, ainsi que des altérations structurelles de l'hippocampe, par l'intermédiaire d'une diminution de la synthèse de BDNF (« *brain-derivated neurotrophic factor* »), d'une sécrétion excessive d'acide glutamique et/ou d'une diminution de la capture du glucose **(6).**

Conformément à ces observations, des inhibiteurs de la synthèse des glucocorticoïdes et des antagonistes des récepteurs de glucocorticoïdes exercent des effets de type AD **(7).**

Des antagonistes agissant sur les récepteurs de la substance P, en particulier le NK1, ou le récepteur CRF (« *corticotropin-releasing factor*»), ainsi que des antagonistes des récepteurs du NMDA ont été développés avec une certaine efficacité **(8-10).**

Diverses études récentes réalisées dans des situations de stress et des modèles de dépression ont impliqué la neurogenèse dans l'étiologie des troubles dépressifs majeurs **(11-12-13).** Il a été démontré que tous les traitements chroniques AD, y compris l'électrochoc, stimulent la prolifération des cellules progénitrices à l'origine des neurones de la couche granulaire de l'hippocampe.

On sait également que les AD modulent l'expression de différents facteurs impliqués dans la survie et la croissance des cellules, tels que la CREB, le Bcl2 ou les MAP-kinases. Toutefois, l'importance fonctionnelle de ces neurones néoformés dans la physiopathologie des troubles de l'humeur reste controversée (14).

L'ensemble de ces indications montrent que la dépression est une maladie complexe dont la physiopathologie est multifactorielle et, en conséquence, le traitement d'une telle pathologie reste un challenge.

Depuis plus de quarante ans, la recherche sur la dépression et le développement de médicaments efficaces a été dominée par l'hypothèse monoaminergique. Bien que les neurotransmetteurs monoaminergiques (sérotonine, noradrénaline et dopamine) sont impliqués de façon indiscutable, le nombre d'hypothèses sur la physiopathologie de la dépression et les mécanismes d'action des AD n'a cessé d'évoluer.

Les médicaments AD utilisés aujourd'hui produisent une gamme d'effets indésirables, parmi lesquels la sécheresse de la bouche, la vision floue et l'altération de la fonction intestinale (diarrhée ou constipation). Même si beaucoup d'effets secondaires sont passagers (comme la nausée), certains semblent être constants au fil du temps (comme les effets sexuels) et risquent d'affecter l'assiduité au traitement à long terme. C'est la raison pour laquelle la recherche de nouvelles molécules agissant sur des récepteurs ou canaux nouvellement identifiés dans la dépression est cruciale.

Certaines protéines, récepteurs et canaux, ont été impliqués dans les mécanismes moléculaires de la dépression. C'est notamment le cas du récepteur 3 de la neurotensine (NTRS3), originellement appelé sortiline, dont l'inactivation chez la souris produit un phénotype de résistance à la dépression, analogue à celui qui a été observé chez des souris invalidées pour le canal potassique de fond TREK-1. Aucune molécule interagissant efficacement avec ces canaux n'a été identifiée à ce jour.

Il existe donc un réel besoin de nouvelles molécules utilisables pour le traitement des troubles psychiatriques, notamment de la dépression, molécules plus efficaces, mieux tolérées et ayant une action plus rapide.

### Exposé de l'invention

La présente invention a précisément pour rôle de répondre au besoin et de résoudre les inconvénients de l'art antérieur.

La maturation du NTSR3 libère un peptide, appelé propeptide, qui devient un ligand de ce même type de récepteur. De façon intéressante, ce propeptide est également capable de bloquer l'activité du canal TREK-1.

En effet, le NTR3-sortiline est synthétisé sous la forme d'un précurseur (proNTR3-sortiline). La maturation de ce précurseur, effectuée par la furine, libère un peptide (propeptide) de 44 acides aminés dont la séquence est la suivante :

QDRLDAPPPPAAPLPRWSGPIGVSWGLRAAAAGGAFPRGGRWRR **SEQ ID N°1**

Ce peptide est capable de se lier au récepteur (NTSR3) maturé. Des études de relations structure-fonction montrent qu'une partie de 17 acides aminés de ce peptide porte toute l'activité de liaison sur le récepteur. Cette partie du peptide est la suivante :

APLPRWSGPIGVSWGLR **SEQ ID N°2**

Cette séquence SEQ ID N°2 correspond à la partie soulignée du grand peptide. C'est ce peptide (que nous avons nommé propeptide) qui a été utilisé dans les expérimentations décrites ci-après.

La présente invention se rapporte donc au peptide de séquence ID N°1 ou 2 ou à un fragment ou à un dérivé de ce peptide qui est un ligand du récepteur 3 de la neurotensine (NTSR3). Les « fragments» et « dérivés » de ce peptide sont ceux que l'homme du métier peut aisément déduire de les séquences ID n°1 ou 2 annexées, par exemple en remplaçant des acides aminés par leur(s) équivalent(s) ou en raccourcissant le peptide sans que son activité en soit modifiée.

Ces « fragments » ou « dérivés » sont tels qu'ils conservent la propriété de ligand du récepteur 3 de la neurotensine (NTSR3) que possède le peptide de la présente invention. Dans la description qui suit, le peptide de l'invention sera appelé « peptide » ou « propeptide ». Les dérivés ou fragment peuvent également être considérés comme étant des analogues du peptide de la présente invention.

Le temps de demi-vie *in vivo* de ce peptide n'est pas encore établi, cependant afin d'améliorer la stabilité ou la biodisponibilité de ce peptide, les inventeurs ont déjà envisagé des modifications sur chacun des acides aminés ainsi que sur la nature des liaisons entre chaque acide aminé. Les modifications sont par exemple les suivantes :
- remplacement d'au moins un acide aminé par un autre de la même famille (aromatique, hydrophobe, basique, etc..) ;
- remplacement d'un acide aminé naturel (amino acide L) par le même acide aminé sous forme D ;
- replacement d'une liaison peptidique entre deux acides aminés par une liaison pseudo-peptidique.

Ces modifications génèrent en effet des peptides qui sont résistants à l'attaque protéolytique des peptidases et protéases.

La présente invention se rapporte également à une séquence d'acide nucléique codant un peptide ou un fragment ou un dérivé de ce peptide. Numéros d'accession GenBank pour les ARNmessagers de la sortiline SORT 1, chez la souris : NM_019972, chez l'Homme : NM_002959. Cette séquence peptidique est par exemple la séquence référencée SEQ ID N°2 sur la liste des séquences annexée. Il peut s'agir de toute séquence appropriée codant le peptide de la présente invention, fragment ou dérivé de celui-ci. Cette séquence est de préférence utilisable pour fabriquer le peptide de la présente invention ou un fragment ou dérivé de celui-ci par transfection.

La présente invention se rapporte également à un vecteur comprenant une séquence d'acide nucléique selon l'invention. Il peut s'agir de tout vecteur approprié pour la transformation d'une cellule hôte en vue de faire fabriquer par ladite cellule, par une technique de recombinaison génétique, le peptide de la présente invention, ou fragment ou dérivé de celui-ci. Le vecteur peut être obtenu à partir d'un vecteur choisi, par exemple, dans le groupe comprenant pIRES et pIRES2 et leurs dérivés, pcDNA3 et ses dérivés, pGEX et ses dérivés.

La présente invention se rapporte donc également à une cellule hôte comprenant un peptide ou un fragment ou un dérivé de ce peptide selon la présente invention et/ou une séquence d'acide nucléique selon la présente invention et/ou un vecteur selon la présente invention. La cellule hôte peut être toute cellule appropriée pour être transformée et fabriquer ledit peptide de l'invention ou fragment ou dérivé de celui-ci. Il peut s'agir par exemple de cellules COS-7, HEK 293 et dérivées, N1E115 et apparentées.

La présente invention se rapporte donc également à un procédé de production d'un peptide ou fragment ou dérivé de celui-ci comprenant les étapes suivantes :
- transfecter une cellule hôte avec un acide nucléique selon la revendication 2 ou transformer une cellule hôte avec un vecteur selon la revendication 3 ;
- cultiver ladite cellule hôte dans des conditions permettant l'expression du peptide de séquence ID N°1 ou fragment ou dérivé de ce peptide ; et
- récupérer ledit peptide de séquence ID N°1 ou fragment ou dérivé de ce peptide.

Les techniques de transfection et de transformation utilisables pour fabriquer le peptide de la présente invention ou fragment ou dérivé de celui-ci sont celles connues de l'homme du métier, par exemples celles décrites dans Alloui A et al, EMBO J. 2006 **(19).**

Selon l'invention, le procédé de fabrication préféré du peptide est la synthèse chimique, par exemple en phase solide. Toute technique connue de l'homme du métier peut être utilisée. Par exemple, le peptide peut être synthétisé selon la technique en phase solide de Krieger DE et al, Proc Nat Acad Sci U.S.A. 1976 **(20).**

La molécule de la présente invention ouvre une nouvelle voie dans la mise en place d'une nouvelle classe d'antidépresseurs et de nouvelles stratégies thérapeutiques des maladies psychiatriques.

La présente invention se rapporte donc également à l'utilisation d'un peptide ou fragment ou dérivé de ce peptide selon l'invention comme médicament.

En particulier, la présente invention se rapporte à l'utilisation d'un peptide ou fragment ou dérivé de ce peptide pour la fabrication d'un médicament destiné au traitement de troubles psychiatriques, par exemple pour la fabrication d'un antidépresseur et/ou d'un anti-douleur.

Le peptide de la présente invention ou fragment ou dérivé de celui-ci, tel que défini ci-dessus, est un produit naturel qui constitue un nouveau type d'antidépresseur. Il permet d'éviter tous les effets secondaires non désirables des médicaments actuellement utilisées pour le traitement des troubles psychiatriques. Les effets secondaires des médicaments utilisés dans l'art antérieur proviennent de leur nature chimique. Ces médicaments de l'art antérieur sont des molécules qui ont souvent la propriété de passer les membranes plasmiques cellulaires de manière passive, ce qui entraîne des interactions non spécifiques avec des effecteurs intracellulaires. Le caractère peptidique ou pseudo-peptidique du peptide de la présente invention permet d'éviter ce problème.

Par ailleurs, l'action directe du peptide de la présente invention sur le canal potassique TREK-1 pourrait bien diminuer les délais d'action, souvent longs d'un antidépresseur classique (voir art antérieur ci-dessus).

En outre, le canal TREK-1 est sensible à l'étirement, à l'osmolarité et à la température. Les inventeurs ont démontré que TREK-1 est l'un des censeurs moléculaires impliqués dans la perception de la douleur. Il est très exprimé dans les neurones sensoriels de petite taille des ganglions de la racine dorsale de la moelle épinière, il est présent à la fois dans les neurones peptidergiques et non peptidergiques et est colocalisé avec TRPV1, un canal ionique non sélectif activé par la capsaïne et impliqué dans l'hyperalgésie thermique. Les souris KO (« knock-out ») sont plus sensibles aux sensations de douleur et à la chaleur. Ce phénotype est localisé sur les fibres-C polymodales, qui sont plus sensibles à la chaleur. Les souris KO sont aussi plus sensibles à des stimuli mécaniques de bas seuil et montrent une hyperalgésie thermique et mécanique accrue en conditions d'inflammation. Ce travail, publié dans Alloui A et al EMBO J. 2006 **(19)** désigne le canal TREK-1 comme une cible très intéressante pour le développement de nouveaux analgésiques. Par conséquent toute molécule active sur ces canaux TREK-1 peut avoir des retombées thérapeutiques importantes dans le domaine de la nociception.

En outre le NTSR3 est impliqué dans l'inflammation. Les inventeurs ont démontré que le NTSR3 était responsable des effets de la neurotensine (NT) sur la migration et la libération de cytokines inflammatoires des cellules microgliales **(21, 22).** Le peptide de la présente invention possède la propriété d'antagoniser les effets de la NT. Par conséquent, toute molécule capable de bloquer les effets pro-inflammatoires de la NT peut avoir des retombées thérapeutiques importantes dans le domaine de l'inflammation cérébrale.

D'autres avantages pourront apparaître à l'homme du métier à la lecture des exemples qui suivent, donnés bien entendu à titre illustratif et non-limitatif en référence aux figures annexées.

### Brève description des figures

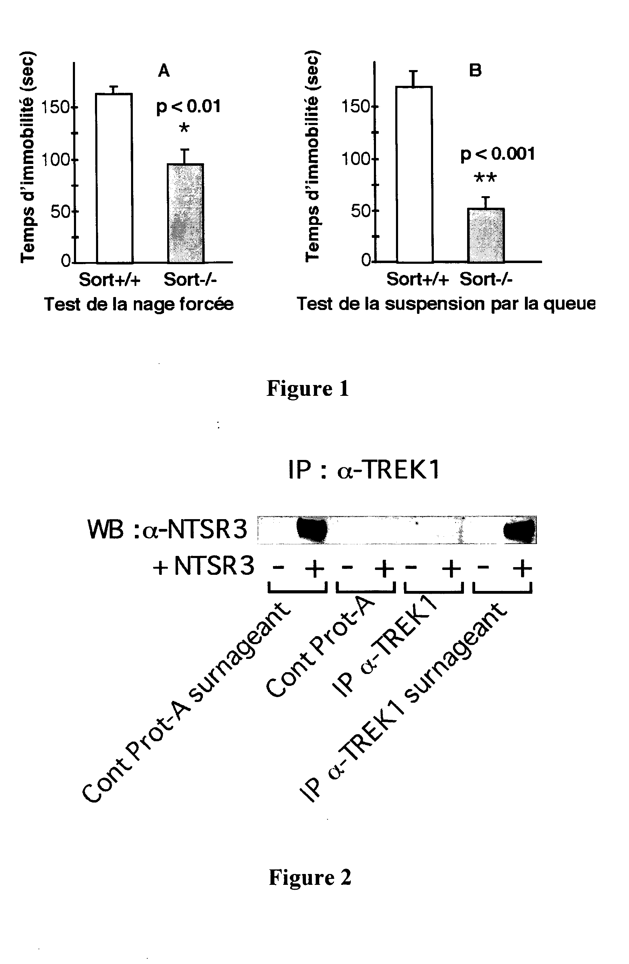

La figure 1 représente des histogrammes rapportant des résultats expérimentaux de tests de résignation (à gauche : test de la nage forcée - à droite, test de suspension par la queue). Sur les histogrammes A et B, en abscisse, le temps pendant lequel l'animal reste immobile (en secondes - sec) et en ordonnée, les différents génotypes des souris testées.

La figure 2 représente un western blot préparé à partir d'extraits membranaires de cellules COS-7 transfectées avec le canal TREK-1 avec ou sans le récepteur NTRS3, immunoprécipités par un anticorps anti-TREK-1 et où les récepteurs NSTR3/sortiline sont révélés à l'aide d'un anticorps anti-sortiline.

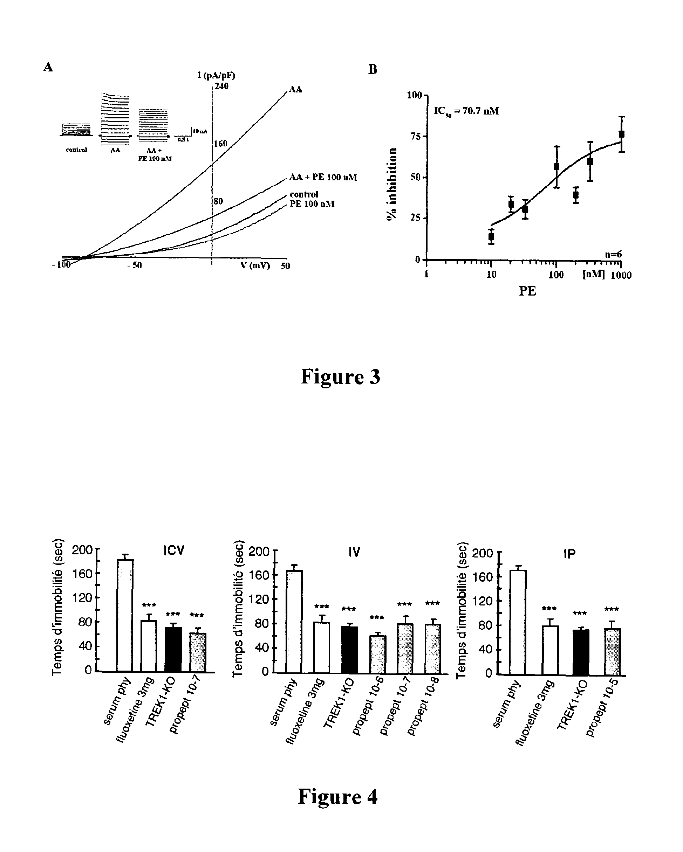

La figure 3 représente des courbes rapportant des résultats expérimentaux de mesures de l'inhibition de l'activité de canaux TREK-1 à 0 mV. Sur le graphique A, en abscisse, est représentée la densité du courant I (pA/pF) et en ordonnée, la tension appliquée (V), exprimée en mV. En insert, les traces représentant l'amplitude du courant aux différentes valeurs des potentiels. Control : conditions de référence, AA, activation du courant par 10µM d'acide arachidonique, AA + PE, activation du courant par 10µM d'acide arachidonique en présence de 100nM de PE. Sur le graphique B, en abscisse, est représenté le pourcentage d'inhibition du courant TREK-1 activé par 10µM d'AA (% inhibition) et en ordonnée, la concentration du PE, exprimée en nM.

La figure 4 représente des histogrammes rapportant des résultats expérimentaux de tests effectués avec trois modes d'injection différents et avec différentes substances injectées. Il s'agit ici également de tests de résignation. Sur les trois graphiques, en abscisse, le temps pendant lequel l'animal reste immobile (en secondes - sec) et en ordonnée, les différentes substances testées.

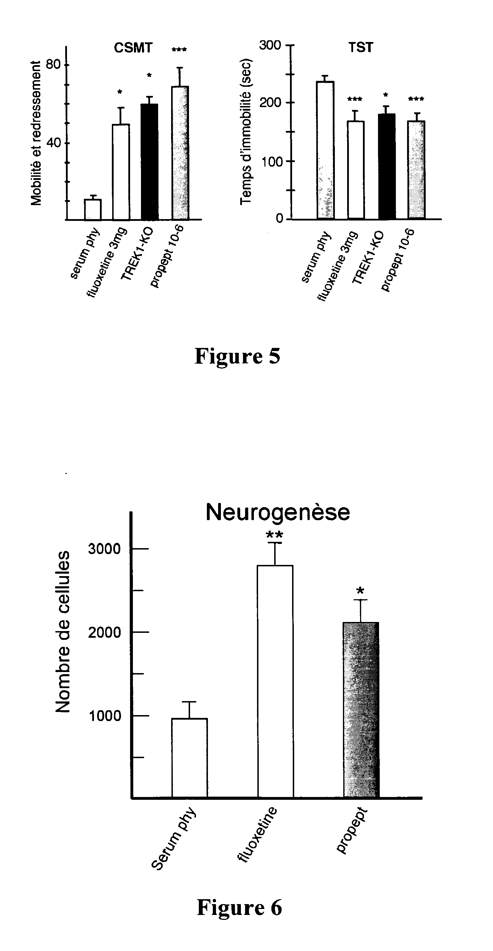

La figure 5 représente des histogrammes rapportant des résultats expérimentaux de test CSMT et TST avec différentes substances injectées. Il s'agit ici également de tests de résignation. Sur le graphique CMST, en abscisse, le nombre de passages d'une case à l'autre et le nombre de redressements et en ordonnée, les différentes substances testées. Sur le graphique TST, en abscisse le temps pendant lequel l'animal reste immobile (en secondes - sec) et en ordonnée, les différentes substances testées.

La figure 6 représente un graphique de mesure de la neurogenèse en présence de sérum physiologique, de fluoxétine ou du peptide de la présente invention. En abscisse, le nombre de cellules positives au marquage BrdU. En ordonnée, les substances testées.

Sur ces figures, pA = picoAmpère et pF = picoFarad.

### EXEMPLES

Les inventeurs ont entrepris des expériences d'électrophysiologie pour démontrer l'inhibition de l'activité des canaux TREK-1 par le propeptide de la présente invention et différents tests dits de comportement visant à valider la propriété antidépresseur du propeptide. En effet, l'équipe du Dr C. Heurteaux a démontré que l'invalidation chez la souris du gène du canal TREK-1 (TREK-1^{-/-} ou KO-TREK-1) conférait aux animaux un phénotype de résistance à la dépression mesuré au travers de tests de comportement reconnus comme étant liés à la dépression. Par conséquent, toute molécule capable soit d'inhiber les canaux TREK-1 soit de reproduire un comportement de type TREK-1^{-/-}, peut être considérée comme étant potentiellement antidépressive.

Les inventeurs ont également réalisé des expériences de colocalisation entre TREK-1 et la sortiline sur des coupes de tronc cérébral de souris.

### Exemple 1 : Essais sur TREK-1

L'équipe de Catherine Heurteaux a mis en évidence que le canal potassique TREK-1 pouvait être une cible pour le traitement de la dépression et que des antagonistes de ce canal pouvaient avoir la propriété d'être des antidépresseurs puissants.

TREK-1 est régulé par les neurotransmetteurs qui modulent le niveau d'AMPc via des récepteurs qui activent la voie des protéines Gq comme le récepteur 5HT (sérotonine). La délétion de TREK-1 résulte en un phénotype anti-dépression avec une augmentation effective de la neurotransmission 5-HT. Les souris KO-TREK-1 développent un comportement similaire à celui de souris naïves traitées avec les anti-dépresseurs classiques. Ces données suggèrent que des bloqueurs du canal TREK-1, qui n'existent pas aujourd'hui, peuvent mener à une nouvelle génération d'anti-dépresseurs. Tout ce qui peut affecter le trafic, l'adressage et la fonction du canal TREK-1 est donc important à identifier. Il s'agit de découvrir des partenaires qui modulent ces propriétés.

Les inventeurs de la présente invention démontrent ici qu'un bon candidat est le récepteur-3 de la neurotensine, le NTSR3 également nommé sortiline. Cette protéine multifonctionnelle peut en effet jouer le rôle de récepteur ou de co-récepteur et elle possède plusieurs ligands tels que la neurotensine, la lipoprotéine lipase et le propeptide libéré lors de la maturation du précurseur du NTSR3/sortiline. Ce propeptide est un antagoniste spécifique des effets de la neurotensine.

Récemment, les inventeurs ont observé que les souris KO-sortiline (sort^{-/-}) présentaient le même comportement que les souris KO-TREK1. Les souris KO-sortiline/NTSR3 ont le même comportement (deux tests de résignation, figure 1 annexée) que les souris traitées avec des anti-dépresseurs classiques. Ils ont donc entrepris des expériences visant à rechercher l'existence d'interactions entre les deux protéines. Pour cela, ils ont réalisé des transfections dans les cellules COS-7 avec les deux protéines NTSR3/sortiline et TREK-1.

Les résultats montrent que l'immuno-précipitation des extraits cellulaires avec un anticorps anti-TREK-1 co-précipite le NTSR3. L'argument le plus direct démontrant une interaction fonctionnelle entre TREK-1 et le NTSR3/sortiline a été obtenu dans des exprériences d'électrophysiologie : l'activation de TREK-1 par l'acide arachidonique est bloquée par le propeptide, un inhibiteur spécifique et sélectif du NTSR3.

Pour obtenir le gel représenté sur la figure 2, les cellules COS-7 transfectées avec TREK-1 et NTSR3/sortiline ont été lysées. Le surnageant a été incubé avec un anticorps anti-TREK-1 (IP : α-TREK-1) en présence de protéine-A sépharose pendant 16 heures à 4°C.

Les protéines ainsi précipitées sont déposées sur un gel SDS-PAGE puis transférées sur nitrocellulose. La protéine NTSR3/sortiline est détectée à l'aide d'un anticorps anti-sortiline.

Les inventeurs de la présente invention démontrent également ici qu'un bon candidat est le récepteur-3 de la neurotensine, le NTSR3 également nommé sortiline. Cette protéine multifonctionnelle peut en effet jouer le rôle de récepteur ou de co-récepteur et elle possède plusieurs ligands tels que la neurotensine, la lipoprotéine lipase et le propeptide libéré lors de la maturation du précurseur du NTSR3/sortiline. Ce propeptide est un antagoniste spécifique des effets de la neurotensine.

### Exemple 2 : Action du propeptide sur l'activité du canal TREK-1

Toutes les expériences de mesure de l'effet du propeptide ont été réalisées sur des cellules COS-7 (lignée cellulaire issue de fibroblastes de rein de singe vert africain Cercopithecus aethiops). Ces cellules sont ensemencées à une densité de 20 000 cellules/ boîte de 35mm de diamètre, 24 heures avant d'être transfectées par la méthode du DEAE-dextran avec 1µg de plasmide pIRES-EGFP-TREK-1. Les mesures de courant sont réalisées 48 à 72 heures après la transfection.

Culture des cellules COS-7. Les cellules COS-7 (référence ATCC : CRL-1651) sont cultivées dans un milieu DMEM (Gibco) / 10% sérum de veau fétal (SVF, ICN), à 37°C en présence de 5% de CO2.

Transfection au DEAE dextran. Au jour 1, les cellules COS-7 sont ensemencées à 20 000 cellules par boîte de diamètre 35mm contenant 2 ml de milieu de culture. Au jour 2, le milieu est éliminé et les cellules sont recouvertes par 200 µl de PBS (Gibco) contenant 1µg de plasmide pIRES-EGFP-TREK-1 et 100 µg de DEAE-dextran (Sigma) puis placées à 37°C/5% CO2. Après 30 minutes d'incubation, on ajoute 2 ml d'un milieu DMEM/ 10% NuSérum/ 80µM chloroquine. Après 3 heures, le milieu est remplacé par 2 ml de DMEM/10% SVF et les cellules sont incubées 48 à 72 heures avant d'être mesurées par des méthodes électrophysiologiques

Mesures électrophysiologiques : toutes les mesures sont faites à température ambiante, c'est-à-dire à 21 - 22°C. Les cellules ayant été transfectées par le plasmide sont repérées grâce à la fluorescence émise par la EGFP après excitation à 480nm.

La technique du patch clamp sur cellule entière a été utilisée pour mesurer l'activité des canaux TREK-1. L'appareillage utilisé est le RK 400 patch-clamp amplifier (Axon Instruments, U.S.A.). Les pipettes de patch de résistance 1,3 à 8 MΩ sont préparées à partir de capillaires en verre borosilicate et sont remplies avec une solution 155 mM KCI, 3 mM MgCl₂, 5 mM EGTA, 10 HEPES/KOH pH 7,2.

Le mileu de culture cellulaire est remplacé par une solution 150 mM NaCl, 5 mM KCI, 3 mM MgCl₂, 1 mM CaCl2, 10 HEPES/ NaOH pH 7,4 contenant 10 mM Chlorure de TétraEthyl Ammonium, 3 mM de 4-Aminopyridine. Les cellules sont perfusées en continu avec cette solution. Le potentiel de repos de la membrane de la cellule mesurée est fixé à -80mV.

Les variations de voltage sont obtenues soit par rampe continue (de - 100 à + 50mV) soit par saut de potentiel de 10mV (de -100 à + 40mV, 1,5 seconde par saut).

Les données obtenues ont été analysées avec le logiciel Pclamp. Les courants décrits dans les figures ont été obtenus à 0 mV, les résultats exprimés sont les moyennes ± la déviation standard. La valeur de l'IC₅₀ a été obtenue en traçant les données expérimentales à l'aide d'une fonction sigmoïdale.

L'activité du canal est ensuite mesurée par la technique de « *patch-clamp* » en configuration cellule entière comme décrit ci-dessus.

Dans des conditions basales, c'est-à-dire en absence d'activation par l'acide arachidonique (connu pour être un activateur puissant du canal TREK-1), 100 µM de propeptide inhibent 25% de l'activité canal mesurée à 0 mV (31 pA/pF contre 23 pA/pF).

Dans les conditions où le canal est activé par 10 µM d'acide arachidonique (31 pA/pF contre 130 pA/pF), 100 µM de propeptide inhibent 67% de l'activité du canal mesurée à 0 mV (130 pA/pF contre 56 pA/pF).

Dans les mêmes conditions expérimentales, une courbe dose-réponse de l'inhibition de l'activité des canaux TREK-1 mesurée à 0 mV a été réalisée et est représentée sur la figure 3 annexée. Elle indique une concentration de demi-effet (IC₅₀) de 70.7 nM.

### Exemple 3 : Tests de comportement

Les inventeurs ont réalisé trois types d'expériences de comportement considérés comme classiques pour déterminer l'activité antidépresseur d'une substance (Nestler EJ et al, 2002 **(15) ;** Cryan J and Holmes A, 2005 **(16).** Les effets du propeptide de la présente invention ont été comparés à ceux de la solution saline dans laquelle il est dissous et ceux de la fluoxétine, un antidépresseur des plus utilisés en clinique. La souche de souris utilisée est la souche C57BIack/j. Des souris TREK-1^{-/-} ont également été mesurées dans ces différents tests.

Le propeptide est dissous dans une solution saline 0,9% NaCl aux concentrations désirées : 10⁻⁵ M, 10⁻⁶ M, 10⁻⁷ M et 10⁻⁸ M. Différents volumes sont injectés en fonction de la technique utilisée :
- en intra cérébro ventriculaire : 5 µl
- en intraveineuse : 100 µl
- en intrapéritonéale : 100 µl

Pour les injections ICV une seringue Hamilton (Fisher Bioblock)(marque de commerce) de 10 µl est utilisée, et dans le cas des injections IV ou IP c'est une aiguille de 0,45 x 12 mm qui est utilisée.

### A. Test de la nage forcée (ou Forced Swimming Test, FST) Cryan & Holmes, 2005 (16))

L'expérience consiste, 30 min après injection de la substance à tester, à plonger la souris pendant 6 minutes dans un récipient de 15 cm de diamètre, de 30 cm de hauteur contenant 11 cm d'une eau à 22°C et de mesurer le temps d'immobilité lors des 4 dernières minutes. Les souris traitées avec des antidépresseurs ont des temps d'immobilité inférieurs à ceux des souris contrôle. Les inventeurs ont testé différentes voies d'injection du peptide et de la solution saline : intra-cérébro-ventriculaire (ICV), intraveineuse (IV) ou intrapéritonéale (IP). Seules des injections IP ont été utilisées pour administrer la fluoxétine. Un contrôle avec une injection de sérum physiologique a été réalisé.

Les résultats de ces tests sont représentés sur la figure 4 annexée : Tests FST, les valeurs ± SEM sont statistiquement comparées à la condition contrôle (sérum physiologique) *** p < 0,001, (Test « ANOVA » = test d'analyse de variances)

Quelle que soit la voie d'administration, les effets du propeptide de la présente invention sont comparables à ceux provoqués par la fluoxétine. Les souris traitées par le propeptide ont un comportement identique aux souris KO-TREK-1.

Dans les deux tests suivants, le propeptide de la présente invention a été administré par voie IV à raison de 100µL à 1µM par animal.

### B. Test de la suspension par la queue (ou « Tail Suspension Test », TST) (Cryan & Holmes, 2005 (16); Ripoll et al, 2003 (17)).

L'expérience consiste, 30 min après injection de la substance testée à la souris, à suspendre la souris par la queue à l'aide d'un ruban adhésif et à mesurer le temps d'immobilité pendant 6 min.

Comme indiqué sur le graphique de la figure 4 annexée (TST), les souris traitées par des antidépresseurs ont des temps d'immobilité diminués par rapport aux souris contrôles.

Dans cette expérimentation encore, les effets du propeptide de la présente invention sont comparables à ceux provoqués par la fluoxétine, et les souris traitées par le propeptide ont un comportement identique aux souris TREK-1^{-/-} (figure 4, TST)).

Le propeptide est dissous dans une solution saline 0,9% NaCl aux concentrations désirées : 10⁻⁵ M, 10⁻⁶ M, 10⁻⁷ M et 10⁻⁸ M. Différents volumes sont injectés en fonction de la technique utilisée :
- en intra cérébro ventriculaire : 5 µl
- en intraveineuse : 100 µl
- en intrapéritonéale : 100 µl

Pour les injections ICV une seringue Hamilton (Fisher Bioblock)(marque de commerce) de 10 µl est utilisée, et dans le cas des injections IV ou IP c'est une aiguille de 0,45 x 12 mm qui est utilisée.

### C. Test de suppression de mobilité conditionnée (ou « Conditioned Motility Suppression Test », CMST) (Daugé et al, 2001, (18))

L'expérience consiste à conditionner, au jour 1, la souris et, au jour 2, mesurer les effets des substances.

Jour 1, chaque souris est placée dans une boîte où le plancher est une grille métallique reliée à un stimulateur électrique. Le conditionnement consiste à générer 30 séquences constituées d'un choc électrique (200ms à 1,8 mA) suivi de 12 secondes de latence (temps total de conditionnement : 6 minutes). Les souris sont ensuite remises en cage et laisser à récupérer sur 24 heures.

Jour 2, les souris sont injectées avec les différentes substances testées puis après 30 minutes, elles sont replacées dans la boîte qui a servi au conditionnement et où le plancher a été divisé en 6 cases identiques. Aucun choc électrique n'est délivré.

La mesure s'effectue en comptant manuellement pendant 6 minutes le nombre de fois où chaque souris change de case ou se redresse.

Les souris choquées non traitées (sérum physiologique) ne bougent pratiquement pas (« *freezing* »), les souris choquées traitées récupèrent une mobilité importante par rapport à ces dernières : 5 fois supérieure avec la fluoxétine (à raison d'une injection de 3 mg/kg) et 7 fois supérieure avec le propeptide de la présente invention.

Les résultats expérimentaux de ce test sont représentés sur la figure 5 annexée.

Les souris TREK-1^{-/-} ont une mobilité 6 fois supérieure au contrôle (CSMT sur la figure 5).

L'ensemble de ces résultats expérimentaux démontrent clairement que le propeptide de la présente invention possède des propriétés d'antidépresseur au moins aussi efficaces que celles de la fluoxétine voire supérieures dans le cas du test conditionnement de suppression de mobilité.

De plus, le fait que le propeptide de la présente invention agisse par voies intraveineuse ou intrapéritonéale aussi bien que par voie intracisternale indique qu'il passe facilement la barrière hémato-méningée (BHE) pour exercer ses effets. Cette propriété ouvre la possibilité de traitements chroniques et facilement transposables pour des tests cliniques.

### Exemple 4 : Effets du propeptide sur la neurogenèse

Les traitements par les antidépresseurs sont connus pour augmenter la neurogenèse dans l'hippocampe, cette prolifération cellulaire est mesurée par l'augmentation de cellules « progénétrices » qui incorporent un marqueur le 5-bromo-2-deoxyuridine (BrdU) et se transforment en neurones matures. La détection du BrdU se fait par immunohistochimie.

Au jour 1, les animaux sont injectés en IP avec 300 NI d'une solution aqueuse de 10mM de BrdU, puis remis en cage.

Au jour 2, les animaux sont euthanasiés puis les tissus sont fixés grâce à la perfusion intracardiaque d'une solution froide de 4% paraformaldéhyde. Les cerveaux sont prélevés puis congelés à -20°C.

Des coupes sériées de 40µm sont préparées, de façon à recouvrir la totalité de la structure hippocampe, à l'aide d'un vibratome (Leica). Une coupe sur six est retenue pour mesurer l'incorporation de BrdU à l'aide d'un anticorps anti-BrdU monoclonal de souris (Becton Dickinson). Cet anticorps primaire est révélé grâce à un anticorps secondaire couplé à la biotine et à de l'avidine-peroxidase. L'activité peroxidase est visualisée par hydrolyse du DAB. Les cellules ainsi marquées sont comptées sur chaque coupe.

Pour chaque groupe, solution saline, propeptide ou fluoxétine, les nombres de cellules marquées au BrdU de chaque coupe sont additionnés et multipliés par six de façon à considérer l'ensemble de la structure. Chaque groupe est constitué de 3 souris, les résultats exprimés sont la moyenne des trois animaux.

Les résultats obtenus sont représentés sur l'histogramme de la figure 6 annexée. Ce graphique indique la mesure de la neurogenèse. Le sérum physiologique (sérum phy) et le propeptide 10µM ont été injectés deux fois par jour pendant 7 jours, la fluoxétine a été administrée par l'eau de boisson, solution à 80 mg/l. **p< 0,01, *p< 0,05.

Ces résultats démontrent clairement que le propeptide induit une neurogenèse importante comparable à celle induite par la fluoxétine. Il n'y aucune différence significative entre les mesures obtenues avec le propeptide et celles obtenues avec la fluoxétine.

### Liste des références

**(1)** Wong, M. & Licinio, J. Research and treatment approaches to depression. Nat Rev Neurosci. 2, 343-351 (2001).
**(2)** Moller HJ. Suicide, suicidality and suicide prevention in affective disorders. Acta Psychiatr Scand; 418 (suppl) : 73-80 (2003).
**(3)** Nestler, E. et al. Neurobiology of depression. Neuron 34, 13-25 (2002).
**(4)** Baghai TC, Volz HP, Moller HJ. Drug treatment of depression in the 2000s: An overview of achievements in the last 10 years and future possibilities.World J Biol Psychiatry; 7(4):198-222 (2006).
**(5)** Weilburg JB. An overview of SSRI and SNRI therapies for depression. Manag Care. Jun; 13 (6 Suppl Depression): 25-337 (2004).
**(6)** Manji HK, Gottesman II, Gould TD. Signal transduction and genes-to-behaviors pathways in psychiatric diseases. Sci STKE; 207 : pe49 (2003).
**(7)** Reus VI, Wolkowitz OM. Antiglucocorticoid drugs in the treatment of depression. Expert Opin Investig Drugs ; 10 : 1789-96 (2001).
**(8)** Griebel G, Simiand J, Steinberg R, et al. 4-(2-Chloro-4-methoxy-5-methylphenyl)-N-[(1S)-2-cyclopropyl-1-(3- fluoro-4- methylphenyl)ethyl]5-methyl-N-(2-propynyl)-1, 3- thiazol-2-amine hydrochloride (SSR125543A), a potent and selective corticotrophin-releasing factor(1) receptor antagonist. II. Characterization in rodent models of stress-related disorders. J Pharmacol Exp Ther; 301 : 333-45 (2002).
(9) Kramer MS, Cutler N, Feighner J, et al. Distinct mechanism for antidepressant activity by blockade of central substance P receptors. Science ; 281 : 1640-1645 (1998).
(10) Skolnick P. Antidepressants for the new millennium. Eur J Pharmacol ; 375 : 31-40 (1999).
(11) Kempermann G, Kronenberg G. Depressed new neurons. Adult hippocampal neurogenesis and a cellular plasticity hypothesis of major depression. Biol Psychiatry ; 54 : 499-503 (2003).
**(12)** Malberg JE, Schecter LE. Increasing hippocampal neurogenesis: a novel mechanism for antidepressant drugs. Curr Pharm Des; 11 : 145-155 (2005)
**(13)** Duman, R. & Monteggia, L. A neurotrophic model for stress-related mood disorders. Biol Psychiatry. (9, 1116-1127 (2006).
**(14)** Henn FA, Vollmayr B. Neurogenesis and depression: etiology or epiphenomenon? Biol Psychiatry; 56 : 146-50.(2004).
**(15)** Nestler, E.J. et al. Preclinical models: status of basic research in depression. Biol Psychiatry. 15, 503-528 (2002).
**(16)** Cryan, J. & Holmes, A. The ascent of mouse: advances in modelling human depression and anxiety. Nat Rev Drug Discov. 4, 775-790 (2005).
**(17)** Ripoll, N., David, D., Dailly, E., Hascoet, M. & Bourin, M. Antidepressant-like effects in various mice strains in the tail suspension. Behav Brain Res. 2003 Aug 14;143(2):193-200. 143, 193-200 (2003).
**(18)** Daugé, V., Sebret, A., Beslot, F., Matsui, T., & Roques B. Behavorial profile of CCK2 receptor-deficient mice. Neuropsychopharmacol. 25 (5), 690-698 (2001).
**(19)** Alloui A, Zimmermann K, Mamet J, Duprat F, Noël J, Chemin J, Guy N, Blondeau N, Voilley N, Rubat-Coudert C, Borsotto M, Romey G, Heurteaux C, Reeh P, Eschalier A, Lazdunski M. TREK-1, a K+ channel involved in polymodal pain perception. EMBO J. 2006 Jun 7; 25(11):2368-76.
**(20)** Krieger DE, Erickson BW, Merrifield RB. Affinity purification of synthetic peptides. Proc Natl Acad Sci U S A. 1976 Sep;73(9):3160-4.
**(21)** Martin S, Vincent JP, Mazella J. Involvement of the neurotensin receptor-3 in the neurotensin-induced migration of human microglia. J Neurosci. 2003 Feb 15;23(4):1198-205.
**(22)** Martin S, Dicou E, Vincent JP, Mazella J. Neurotensin and the neurotensin receptor-3 in microglial cells. J Neurosci Res. 2005 Aug 1 ;81 (3):322-6.

## Revendications

1. Peptide de séquences ID N°1 ou 2 ou un fragment ou un dérivé de ce peptide qui est un ligand du récepteur 3 de la neurotensine (NTSR3).

2. Séquence d'acide nucléique codant un peptide ou un fragment ou un dérivé de ce peptide selon la revendication 1.

3. Vecteur comprenant une séquence d'acide nucléique selon la revendication 2.

4. Cellule hôte comprenant un peptide ou un fragment ou un dérivé de ce peptide selon la revendication 1 et/ou une séquence d'acide nucléique selon la revendication 2 et/ou un vecteur selon la revendication 3.

5. Procédé de production d'un peptide ou fragment ou dérivé de celui-ci selon la revendication 1 comprenant les étapes suivantes :
- transfecter une cellule hôte avec un acide nucléique selon la revendication 2 ou transformer une cellule hôte avec un vecteur selon la revendication 3 ;
- cultiver ladite cellule hôte dans des conditions permettant l'expression du peptide ou fragment ou dérivé de ce peptide selon la revendication 1 ; et
- récupérer ledit peptide ou fragment ou dérivé de ce peptide.

6. Utilisation d'un peptide ou fragment ou dérivé de ce peptide selon la revendication 1 comme médicament.

7. Utilisation d'un peptide ou fragment ou dérivé de ce peptide selon la revendication 1 pour la fabrication d'un médicament destiné au traitement de troubles psychiatriques, de phénomènes inflammatoires et/ou douloureux.

8. Utilisation selon la revendication 7, pour la fabrication d'un antidépresseur.

## Revendications modifiées

### Revendications modifiées conformément à la règle 137(2) CBE.

**1.** Peptide de séquences ID N° 2 ou un fragment ou un dérivé de ce peptide qui est un ligand du récepteur 3 de la neurotensine (NTSR3).

**2.** Séquence d'acide nucléique codant un peptide ou un fragment ou un dérivé de ce peptide selon la revendication 1.

**3.** Vecteur comprenant une séquence d'acide nucléique selon la revendication 2.

**4.** Cellule hôte comprenant un peptide ou un fragment ou un dérivé de ce peptide selon la revendication 1 et/ou une séquence d'acide nucléique selon la revendication 2 et/ou un vecteur selon la revendication 3.

**5.** Procédé de production d'un peptide ou fragment ou dérivé de celui-ci selon la revendication 1 comprenant les étapes suivantes :
- transfecter une cellule hôte avec un acide nucléique selon la revendication 2 ou transformer une cellule hôte avec un vecteur selon la revendication 3 ;
- cultiver ladite cellule hôte dans des conditions permettant l'expression du peptide ou fragment ou dérivé de ce peptide selon la revendication 1 ; et
- récupérer ledit peptide ou fragment ou dérivé de ce peptide.

**6.** Utilisation d'un peptide ou fragment ou dérivé de ce peptide selon la revendication 1 pour la fabrication d'un médicament destiné au traitement de troubles psychiatriques, de phénomènes inflammatoires et/ou douloureux.

**7.** Utilisation selon la revendication 6, pour la fabrication d'un antidépresseur.


SEQUENCE LISTING

<110>  Centre National de la Recherche Scientifique (CNRS)
       MAZELLA, Jean
       HEURTEAUX, Catherine
       BORSOTTO, Marc
       PETRAULT, Olivier
       WIDMANN, Catherine

<120>  PEPTIDE DERIVE DE NSTR3 ET UTILISATION DANS LE TRAITEMEMENT DE
       MALADIES PSYCHIATRIQUES

<130>  103065/EP

<160>  2

<170>  PatentIn version 3.3

<210>  1
<211>  44
<212>  PRT
<213>  Homo sapiens

<400>  1

Gln Asp Arg Leu Asp Ala Pro Pro Pro Pro Ala Ala Pro Leu Pro Arg
1               5                   10                  15


Trp Ser Gly Pro Ile Gly Val Ser Trp Gly Leu Arg Ala Ala Ala Ala
            20                  25                  30


Gly Gly Ala Phe Pro Arg Gly Gly Arg Trp Arg Arg
        35                  40


<210>  2
<211>  17
<212>  PRT
<213>  Homo sapiens

<400>  2

Ala Pro Leu Pro Arg Trp Ser Gly Pro Ile Gly Val Ser Trp Gly Leu
1               5                   10                  15


Arg